**Europäisches Patentamt**

**European Patent Office**

**Office européen des brevets**

(19)

(11) Veröffentlichungsnummer : **0 603 115 A2**

## EUROPÄISCHE PATENTANMELDUNG

(12)

(21) Anmeldenummer : **93810782.8**

(22) Anmeldetag : **11.11.93**

(51) Int. Cl.$^5$ : **A61B 5/103**

(30) Priorität : **10.12.92 US 988699**

(43) Veröffentlichungstag der Anmeldung :
**22.06.94 Patentblatt 94/25**

(84) Benannte Vertragsstaaten :
**CH DE FR IT LI**

(71) Anmelder : **K.K. HOLDING AG**
**Postfach 304**
**CH-8408 Winterthur (CH)**

(72) Erfinder : **Fuglewicz, Daniel P.**
**16 Banko Drive**
**Depew, New York 14043 (US)**
Erfinder : **Schieb, David A.**
**303 Overbrook Avenue**
**Tonawanda, New York (US)**
Erfinder : **Sonderegger, Conrad**
**60 Creekside Drive**
**Tonawanda, New York (US)**

(54) **Tretmühle für Ganganalyse.**

(57)   Ein Fussaufschlagmesssystem und Verfahren, das ein Paar Tandemplatten verwendet, jedes mit mindestens zwei Sensoren enlang der Gehrichtung, um die Kraft zu messen. Durch die Ueberwachung der Signale an den anstossenden Kanten und den Vergleich mit denjenigen an den nichtanstossenden Kanten bestimmt das System ob ein Fuss auf beiden Platten oder ein Fuss auf jeder Platte steht. Dies ermöglicht die getrennte Erfassung und Verfolgung der Sensorsignale für jeden Fuss. Unter Anwendung der Tretmühlengeschwindigkeit wird das Druckzentrum ebenfalls berechnet.

Abb. 1a

EP 0 603 115 A2

Technisches Gebiet

Die vorliegende Erfindung bezieht sich allgemein auf Gang- sowie posturographische Analyse, und spezifischer auf eine Messtechnik, die Signale für die dynamische Analyse vom Gehen und Gleichgewicht liefert.

Hintergrundtechnik

Die Ganganalyse ist ein wichtiges klinisches sowie Forschungsinstrument für die Auswertung und Diagnose von Gangnormen und -Abnormalitäten. Zur Bestimmung der Bodenreaktionskräfte werden allgemein Kraftplatten verwendet. Die Analyse schliesst Parameter ein, z.B. eine Zeitkurve der Bodenreaktionskraft, Zeit und Grösse der ersten und zweiten Spitzenkräfte, Belastungs- sowie Entlastungsraten, Kraftvektoranzeige, Zentrum der Druckwege, und andere der individuellen Gangart bezüglichen Parameter.

Ueblicherweise werden eine oder mehrere Kraftplatten entlang einem festen Gehweg angeordnet, wie im U.S.-Patent 3 894 437 gezeigt. Solche Gehwege sind typischerweise 1.5 m breit x 10 m lang. Als Alternative zu einer fixen Gehweglänge wurden Tretmühlen zur Ganganalyse verwendet. Die Tretmühlen enthalten entweder eine einzige fixe Platte zur Ueberwachung der Kräfte sowohl des rechten sowie des linken Fusses während des Aufsetzens, oder zwei Kraftplatten parallel zur Gangrichtung unter entweder einer einzigen Tretmühle oder Riemen oder zwei Parallelriemen. Jede Kraftplatte überwacht das Aufsetzen auf die jeweilige Platte.

Der Nachteil der Einzelplatte besteht darin, dass es zwischen dem rechten und linken Fuss nicht unterscheiden kann, noch dem gleichzeitigen Aufsetzen beider Füsse auf eine einzige Kraftplatte. In Abständen einem Gehweg entlang angeordnete Platten erfassen einen gleichmässigen Schritt oft nicht, nämlich die Entfernung zwischen dem Aufsetzen des rechten und linken Fusses oder die Gangzyklen rechts-links-rechts. Die Fähigkeit, von einer Person normale Gangmuster zu erfassen wird dadurch beeinträchtigt, dass die Person das Aufsetzen auf die in einer bestimmten Anordnung verlegte Platte bezw. Platten achten muss. Dies wird als Aufsetzbeeinflussung bezeichnet.

Auch gestattet ein Gehweg keine Ganganalyse bei verschiedenen fixen, kontrollierten und genauen Geschwindigkeiten. Veränderlichkeit der Gangparameter resultiert aus den unterschiedlichen Gehgeschwinigkeiten.

Wie der Gehweg erlaubt die Einzelplattentretmühle keine Unterscheidung des einzelnen Fussaufsetzens, wenn sich zwei Füsse gleichzeitig auf der Tretmühle befinden. Deshalb sind Einzelplattentretmühlen nur für Laufschrittanalysen genau, da in diesem Fall nur ein Fuss aufs Mal aufsetzt. Die Tretmühle mit zwei Paralellplatten wiederum ruft eine unnatürliche Gangart hervor, und damit Aufsetzbeeinflussung.

Zusammenfassung der Erfindung

Ziel der vorliegenden Erfindung also ist die Schaffung eines Messsystems für das Fussaufsetzen, das mit einer Tretmühle oder einem Gehweg verwendbar ist und zwischen den einzelnen Fussaufsetzen unterscheiden kann.

Ein weiteres Ziel der vorliegenden Erfindung ist die Schaffung eines Messsystems für das Fussaufsetzen, das ohne Aufsetzbeeinflussung ist.

Ein weiteres Ziel der vorliegenden Erfindung ist die Schaffung eines Messsystems für das Fussaufsetzen, des wiederholte Messungen des ganzen Fussaufsetzens berücksichtigt.

Diese und andere Ziele werden durch ein Messsystem für das Fussaufsetzen erreicht, die erste und zweite Platten hintereinander gegenüber der Gehrichtung der Person auf dem System angeordnet hat. Erste und zweite Sensorsysteme erfassen die Aufsetzkräfte auf den entsprechenden Platten. Von den ersten und zweiten Sensoren bestimmt das System, ob ein einzelner Fuss auf beiden Platten steht, oder je ein Fuss auf jeder Platte. Die Signale werden von den ersten und zweiten Sensoren entsprechend der Bestimmung von einem Fuss auf beiden Platten oder einem Fuss auf jeder Platte verarbeitet, um die individuellen Fussaufsetzen zu messen. Das Sensorsystem für jede Platte umfasst mindestens zwei, entlang der Gehrichtung in Abständen angeordnete Messwandler. Das Aufsetzen eines einzelnen Fusses auf beide Platten wird festgestellt, wenn die Summe der Kräfte an einem ersten Messwandlerpaar, an je einer nebeneinanderliegenden Platte, die Summe der Kräfte an einem zweiten Messwandlerpaar übersteigt, an je einer Platte und von dem ersten Messwandlerpaar getrennt. An jeder Platte in Abständen entlang der Gehrichtung befinden sich zwei zusätzliche Messwandler, angeordnet quer zur Gehrichtung von einem entsprechenden ersten Messwandler entfernt.

Ein Druckzentrum für jedes Fussaufsetzen wird aus den Kräften auf das erste und zweite Sensorpaar an beiden Platten bestimmt. Ueber den Platten befindet sich ein Tretmühlenriemen, mit einem Sensor zur Bestimmung der Riemengeschwindigkeit. Die Geschwindigkeit dient zur Bestimmung des Druckzentrums für jedes Fussaufsetzen. Die Tretmühlengeschwindigkeit wird geregelt, um die Testperson wesentlich zentriert über den Platten entlang der Gehrichtung zu halten. Falls notwendig werden die Sensoren für jede Platte individuell rückgestellt, wenn in einem Messzyklus keine Kraft von einem Sensor erfasst wird. Signale von dem ersten und zweiten Sensorsystem werden kombiniert, wenn ein Fuss bestimmt wird, und die Signale werden getrennt, wenn ein Fuss auf jeder Platte bestimmt wird.

Die Methode der individuellen Signalverarbeitung für fortlaufendes Fussaufsetzen in einem Zweiplattentandemsystem umfasst die Bestimmung aus ersten und zweiten Sensoren unter der entsprechenden Platte, wenn die Kraft auf die beiden Platten näher zu anstossenden oder nichtanstossenden Kanten der Platten entlang der Gehrichtung sich befindet. Signale von dem ersten und zweiten Sensor werden für ein einzelnes Fussaufsetzen kombiniert, wenn die Kraft als nahe den anstossenden Kanten bestimmt wird. Wird die Kraft näher den nichtanstossenden Kanten bestimmt, werden die Signale als Fussaufschlagpaar getrennt.

Weitere Ziele, Vorteile sowie neuartige Eigenschaften der vorliegenden Erfindung werden aus der folgenden Detailerklärung bei Betrachtung im Zusammenhang mit den zugehörigen Skizzen hervortreten.

## Kurzbeschreibung der Skizzen

Abbn. 1A und 1B zeigen seitliche bezw. Plansichten einer Tretmühle, welche die Grundsätze der vorliegenden Erfinding verkörpert.

Abb. 2 zeigt ein Blockdiagramm eines Systems, das die Grundsätze der vorliegenden Erfindung verkörpert.

Abbn. 3A-3C zeigen die Lagen des linken bezw. rechten Fusses einer Person gegenüber den Kraftplatten der vorliegenden Erfindung.

Abbn. 4A und 4B sind Planansichten den Abbn. 3B und 3C entsprechend.

Abbn. 5A und 5B sind Aufzeichnungen der an der ersten bezw. zweiten Kraftplatte gemessenen Kräfte.

Abb. 5C zeigt den Verlauf der kombinierten Signale von Abbn.

5A und 5B.

Abbn. 5D und 5E zeigen wiederkombinierte Signale des linken bezw. rechten Fusses, den Grundsätzen der vorliegenden Erfindung entsprechend.

Abb. 6 zeigt die Summe der Kräfte in drei orthogonalen Achsen eines einzelnen Fussaufsetzens, durch die Sensoren erfasst.

Abbn. 7A und 7B zeigen den Verlauf der von den vier Messwandlern der ersten bezw. zweiten Platte aufgenommenen Kräfte.

Abb. 7C zeigt den Verlauf der Summe von den Messwandlern I, II, VI, VIII und den Messwandlern III, IV, V, VI.

Abb. 8 ist die Aufzeichnung des gemäss den Grundsätzen der vorliegenden Erfindung berechneten Druckzentrums.

## Günstigste Ausführung der Erfindung

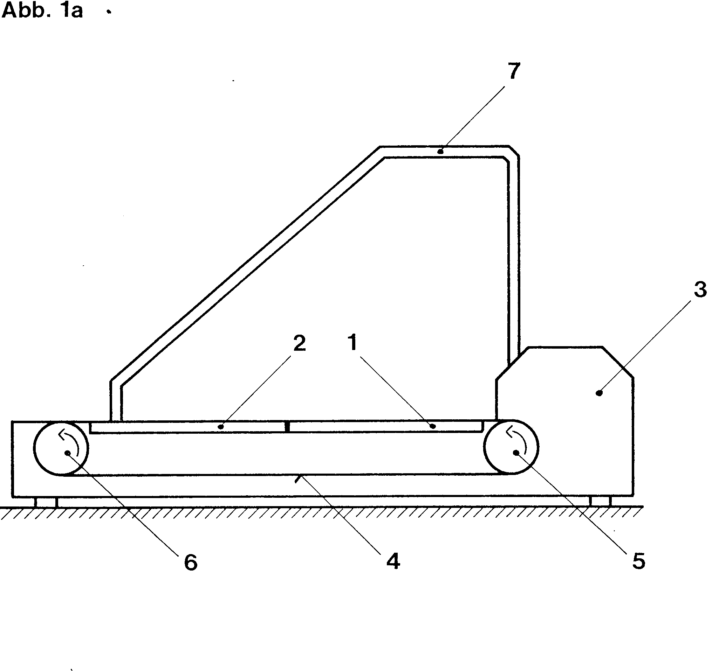

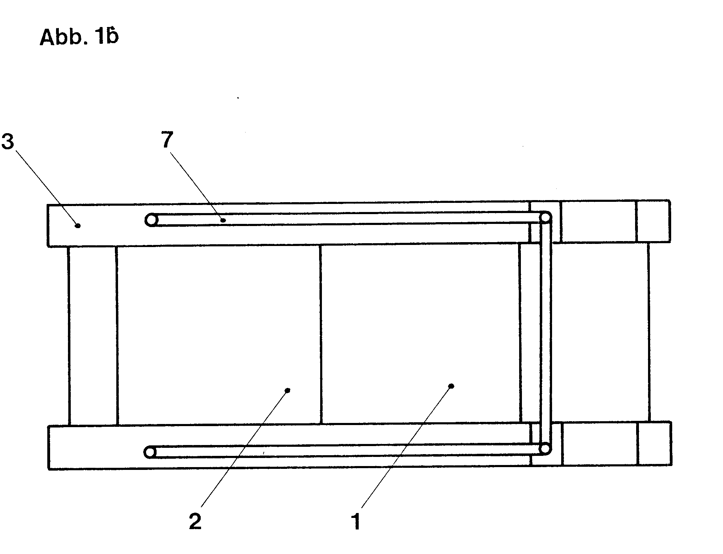

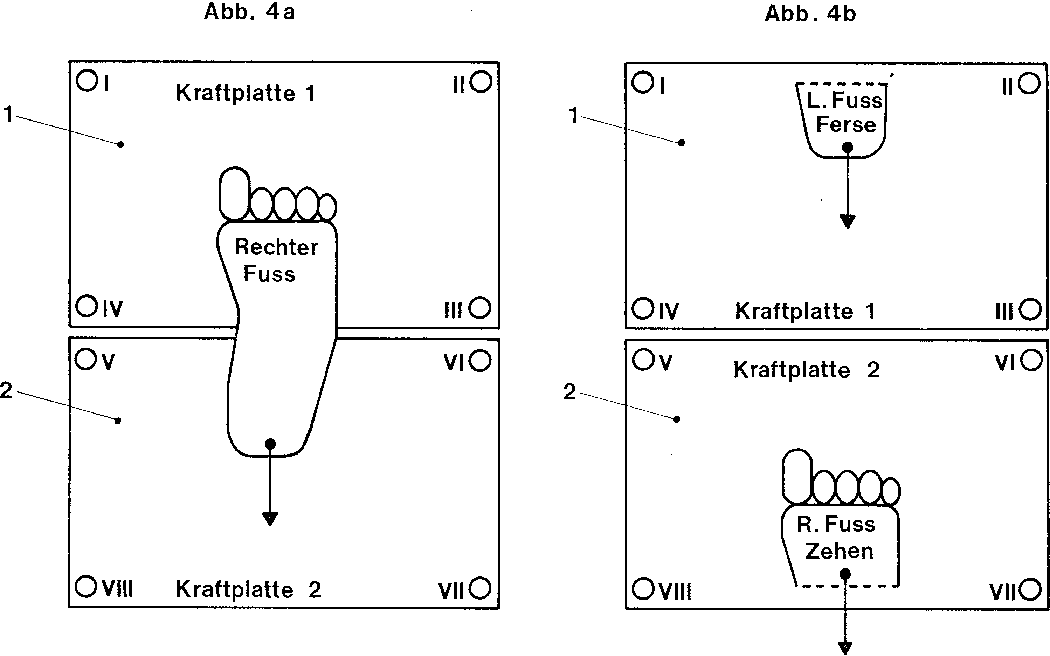

In einer ersten Ausführung gemäss Abbn. 1A und 1B kombiniert eine Tandemkraftplattentretmühle die Messfähigkeiten eines mit Kraftplatten instrumentierten Gehweges mit dem Konfort der Tretmühlentechnik für die Messung von Bodenreaktionskräften für Gang- sowie posturographische Analysen. Das Kraftplattenpaar 1 und 2 wird hintereinander entlang der Laufrichtung unter einem Tretmühlenriemen 4 angeordnet. Ein Paar rotierende Trommeln 5 und 6 werden an dem Rahmen 3 befestigt und treiben den Tretmühlenriemen 4. An dem Rahmen 3 befindet sich noch ein Handgeländer 7. Die Kraftplatten 1 und 2 umfassen Sensorsysteme, die Kräften in einer oder mehreren Achsen empfindlich sind. Diese Sensoren werden noch spezifischer beschrieben, in Bezug auf Abbn. 4A und 4B.

Wie unten besprochen wird, ermöglicht die Tandemanordnung der Kraftplatten 1 und 2 in der Laufrichtung eine Unterscheidung zwischen dem Aufsetzen des linken und rechten Fusses durch das System, wenn eine Person auf dem Riemen geht. Da des System in der Tretmühlenkonstruktion integriert ist, wird sehr wenig Platz beansprucht und die Testperson bleibt während des Gehens in der gleichen Relativlage. Synergistische Messungen wie z.B. Sauerstoffaufname, EKG, EMG, EEG sowie kinematische Messungen können bequem durchgeführt werden, ohne die Beschränkungen von beweglicher Instrumentierung, panoramierenden Kameras oder übermässiger Kablierung.

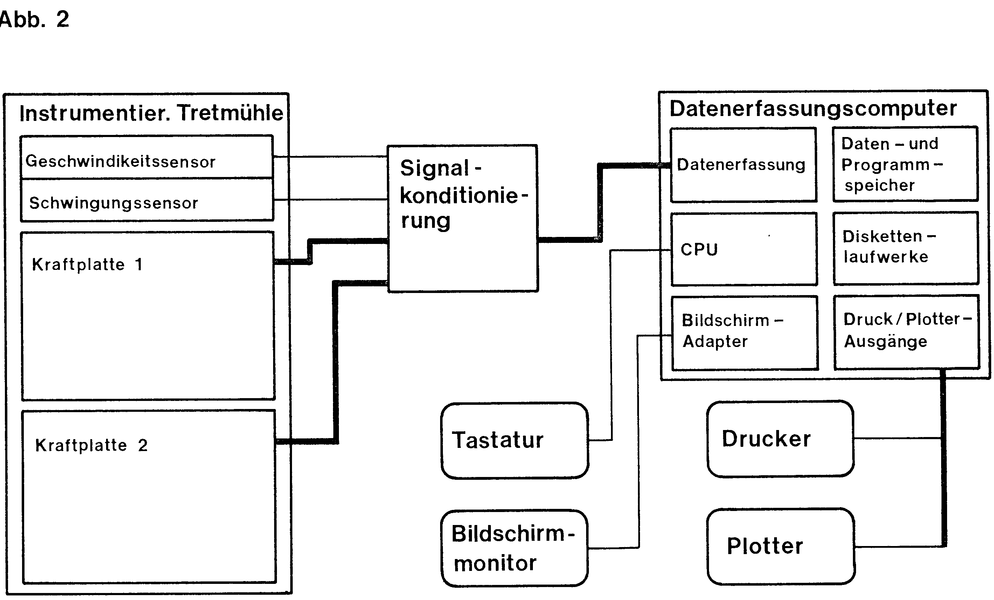

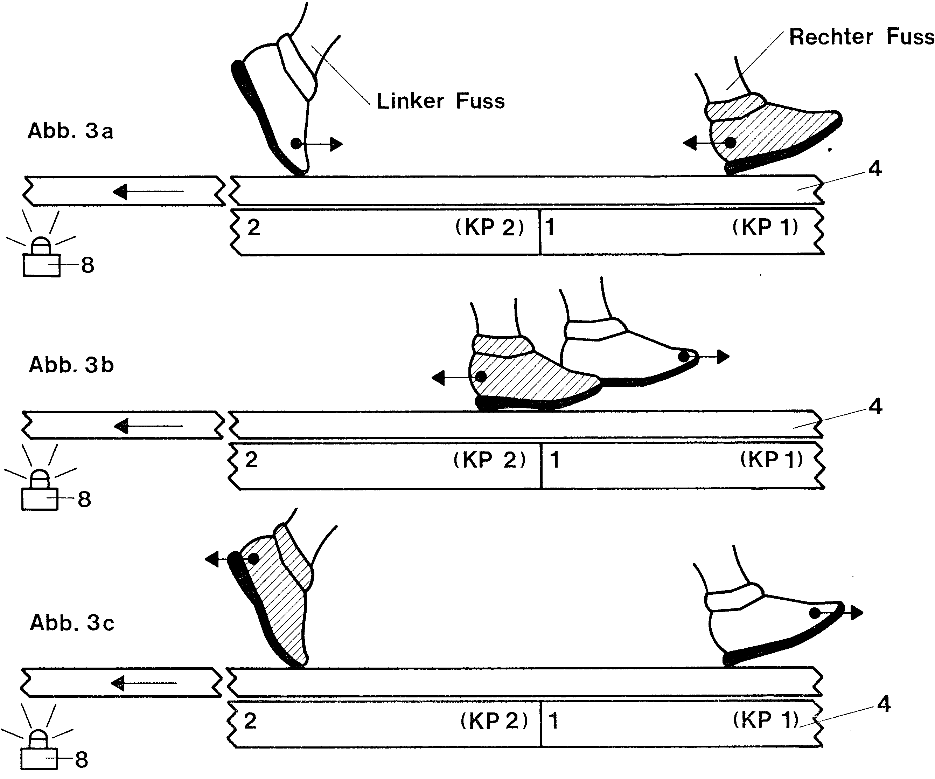

Das in Abb. 2 gezeigte Instrumentierungssystem weist eine Tretmühle mit Kraftplatten 1 und 2 sowie einen Geschwindigkeitssensor auf. Ebenfalls vorhanden ist ein fakultativer Schwingungssensor. Der Ausgang von den Kraftplatten erfolgt durch Signalkonditionierern (z.B. Verstärkung und/oder Filtrierung) an das Datenerfassungssystem. Die Geschwindigkeits- und Schwingungssensoren werden ebenfalls an das Datenerfassungssystem angeschlossen. Das Datenerfassungssystem wandelt die Analogsignale in Digitalsignale um. Daten- und Programmspeicher, Diskettenlaufwerke, Bildschirmadapter, Drucker/Plotterausgänge können versehen werden, um einen entsprechenden Bildschirmmonitor, Drucker bezw. Plotter zu treiben. Ebenfalls vorhanden ist eine Tastatur für die Eingangssteuerung des Datenerfassungscomputers. Der Datenerfassungscomputer erfasst und reduziert Daten, und zeigt die Kräfte sowie dazugehörige Parameter während der Datenerfassung an. Der Schwingungssensor ermöglicht dem Datenerfassungssystem eine zusätzliche Signalverarbeitung, um Systemschwingungssignale aus den Signalen von der Kraftsensorplatte zu entfernen. Ein in Abb. 3A gezeigte Geschwindigkeitssensor wird in dem Datenerfassungssystem verwendet, um das Druckzentrum zu berechnen, wie weiter unten erläutert wird. Die Riemengeschwindigkeit gibt ebenfalls die Gehgeschwindigkeit. Die rotierenden Trommeln 5 und 6 werden drehzahlgesteuert. Die Testperson bleibt über den beiden Kraftplatten 1 und 2 wesentlich zentriert.

Zu den Rauschquellen im Signal zählen Hochfrequenzrausch von dem Motor sowie Störungen durch den Riemenschlupf an den Platten. Periodischer Rausch lässt sich z.B. durch Digital-filtrierung eliminieren.

Die allgemeine Funktion des Systems wird mit Bezug auf Abbn. 3 und 4 erläutert, wobei der linke Fuss LF und der rechte Fuss RF der Testperson in verschiedenen Lagen auf den Kraftplatten gezeigt werden. Die Pfeile zeigen die Richtung der Fussbewegung. In Abb. 3A befindet sich der linke Fuss der Testperson hinter dem rechten und wird von der Zehe angehoben. Der das Körpergewicht tragende rechte Fuss befindet sich über der ersten Kraftplatte P1 und wird durch den Riemen 4 rückwärts bewegt. In Abb. 3A also befindet sich nur ein Fuss auf jeder Platte. Es wird als ein Endpunkt des Schritts betrachtet. In Abb. 3B befindet sich die Testperson nahe der Mittelauflage, wobei der rechte Fuss durch der Tretmühlenriemen bewegt wird, um auf den beiden Kraftplatten 1 und 2 zu liegen. In Abb. 3C beginnt ein neuer Schritt mit dem Aufsetzen des linken Fusses auf die Kraftplatte 1 mit der Ferse zuerst, wobei der rechte Fuss von der Zehe weg von der Kraftplatte 2 abgehoben wird. Dies ist der zweite Endpunkt für den ersten Schritt, mit der Ferse des linken Fusses auf der einen Kraftplatte 1 und der Zehe des rechten Fusses auf der zweiten Kraftplatte 2. Danach wiederholt sich der Zyklus, wie in Abb. 3A beginnend, nur mit den beiden Füssen umgestellt. Also wird der rechte Fuss von der Kraftplatte 2 angehoben, während sich der linke Fuss rückwärts von der Kraftplatte 1 bewegt um die Platten 1 and 2 zu überbrücken und schliesslich die Platte 2 vollständig zu überqueren.

Wie aus der Erläuterung zu den Abbn. 3A bis 3C ersichtlich wird, gibt es Zeiten, wo die Kraftplatten 1 und 2 die Signale von einem einzigen Fuss kombiniert erfassen, sowie andere Zeiten, wo sie Signale von beiden Füssen empfangen. Obwohl die Signale von den beiden Kraftplatten natürlich getrennt sind, muss das Datenerfassungssystem zwischen der Mittelpunktlage der Abb. 3B unterscheiden, wo ein Fuss auf beiden Kraftplatten steht, und der Lage in Abbn. 3A und 3C, wo ein Fuss auf jeder Platte steht. Wie weiter unter erläutert wird, ist das Datenerfassungssystem in Abb. 2 imstande, eine solche Unterscheidung zu vollziehen, und es erfasst und koordiniert die Kraftplattensignale für jeden Fuss individuell und zeitlich kontinuierlich.

Die Kraftplatte 1 besitzt vier Sensoren bezw. Messwandler I, II, III und IV, während die zweite Kraftplatte vier Sensoren bezw. Messwandler V, VI, VII und VIII hat. In den vier Ecken der Kraftplatte werden Sensoren versehen. Jede Kraftplatte umfasst zwei Sensorpaare entlang der Laufrichtung verteilt und voneinander quer zur Laufrichtung gesperrt. Sensoren IV und V sowie III und VI befinden sich an anstossenden Kanten, während Sensoren I und VII sowie II und VII gelten als Sensoren an nichtanstossenden Kanten. Diese werden weiter unten besprochen im Zusammenhang mit der Bestimmung, ob sich ein Einzelfuss über beide Kraftplatten erstreckt oder zwei Füsse auf einer Platte stehen. Die vier Messwandler an einer Platte messen die Kraft in einer oder mehreren Richtungen bezw. Achsen. Zu den verwendbaren Fühlelementen zählen piezoelektrische, Dehnungsmessfühler, Kondensatoren, Druck, induktive, piezoelektrische Folien oder sonstige Vorrichtungen oder Systeme, um eine Kraftmessung zu vollziehen bezw. ableiten.

Eine Einachsenkraftplatte, einschliesslich Kraftsensoren an den vier Ecken, ist Stand der Technik, z.B. Modell Z15506 von Kistler. Dies ist eine 40 x 40 cm Platte. Gemäss unserer vollständigen Analyse des Gangs sowie gangbezogener Funktionen lässt sich eine Dreiachsenkraftplatte verwenden, z.B. Modell-Nr. 9281B11 von Kistler. Dies ist eine 40 x 60 cm Mehrkomponenten-Kraftplatte.

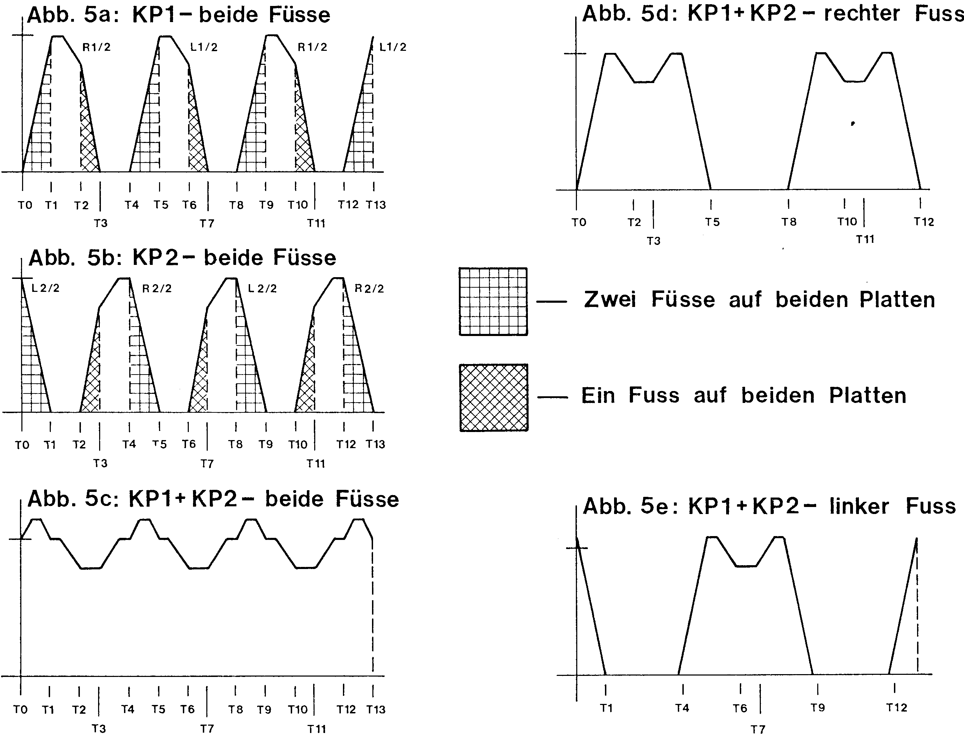

Abbn. 5A bis 5E zeigen die an jeder von den Kraftplatten empfangenen Signale. Abb. 5A zeigt die Kräfte an Platte FP1, während Abb. 5B die Kräfte an Platte FP2 zeigt. Von der Zeit T0 bis T3 befindet sich der rechte Fuss auf Kraftplatte 1. Von T0 bis T1 liegt die Ferse des rechten Fusses auf Platte 1 (Abb. 5A), während die Zehe des linken Fusses auf Platte 2 steht (Abb. 5B). Dies ist der Zustand von Abb. 3A. Zu T1 ist die linke Zehe weg von Platte 2. Zu T2 erreicht der rechte Fuss die Schrittmitte oder Mittelauflage und beginnt von Platte 1 zu Platte 2 zu übergreifen. Diese Ueberschneidungszeit erstreckt sich von T2 zu T3. Abbn. 3B und 4A zeigen die Zeitspanne von T2 bis T3. Die Kreuzschattur in diesen beiden Diagrammen zeigt die zeitlichen Ueberschneidungen sowie die Anzahl der getragenen Füsse. Von T3 bis T5 ist der rechte Fuss weg von der Kraftplatte 1 and nu auf der Platte 2. Als die Testperson das Ende des in Abbn. 3C und 4B gezeigten ersten Schittes erreicht, beginnt der rechte Fuss mit dem Zehen abzuheben von der Zeit T4 bis T5 auf Platte 2, während die Ferse des linken Fusses auf die Platte 1 setzt wie zu den Zeiten T4 bis T5 gezeigt wird. Darauf wiederholt sich der Zyklus für den linken und rechten Fuss.

Die kombinierten Signale von Kraftplatten 1 und 2 in Abb. 5A und 5B würden bei Verwendung einer einzigen Kraftplatte das in Abb. 5C wiedergegebene Signal ergeben. Das Ziel besteht darin, die Signale für den linken und rechten Fuss von den beiden Kraftplatten wie in Abbn. 5A und 5B gezeigt zu trennen, um unabhängige Signale für den rechten und linken Fuss zu erhalten. Die Signalverarbeitung gemäss der vorliegenden Erfindung erzeugt die unabhängigen Signale für den rechten Fuss in Abb. 5D und für den linken Fuss in Abb. 5E. Das Signal für den rechten Fuss in Abb. 5D erstreckt sich von der Zeit T0 bis T5, unter Anwendung der Signale von der ersten Kraftplatte von T1 bis T3, sowie derjenigen von der zweiten Platte von T2 bis T5. Der zweite Rechtsfuss wird gezeigt als von T8 bis T13 stattfindend. Für den linken Fuss erstreckt sich das Signal von T4 bis T9. Das Signal von der ersten Kraftplatte 1 wird für die Zeit von T4 bis T7 verwendet, dasjenige von der zweiten Platte 2 von T6 bis T9.

Damit werden z.B. für die Zeit von T2 bis T3 die Signale von den beiden Kraftplatten summiert, da sie einem gemeinsamen rechten Fuss entstammen. Während der Ueberschneidungszeit T4 bis T5 geben beide Platten Signale, aber sie stammen von verschiedenene Füssen und müssen deshalb getrennt werden. Das vorliegende System bietet ein Mittel zur Bestimmung, ob die Signale von den beiden Platten von einem gemeinsamen Fuss und deshalb summiert sind, oder von einem separaten Fuss auf jeder Platte, unter Anwendung lediglich der Signale von den Kraftplatten.

Das elektronische Signalverarbeitungssystem kann einen gewissen Signaldrift über die Zeit aufweisen. Durch die Verwendung der Tandemkraftplatten 1 und 2 lässt sich jeder Drift während der Signalerfassung eliminieren. Spezifischer, auf Abbn. 5A und 5B bezogen, hat jede Platte eine Periode in der sie kein Signal abgibt. In Abb. 5A z.B. hat Platte FP1 keinen Ausgang zwischen T3-T4, T7-T8 und T11-T12. Analog dazu gibt Platte FP2 in Abb. 5B keine Drucksignale zwischen T1-T2, T5-T6 und T9-T10. Der Signalvorverarbeiter bezw. Datenerfassungssystem mag feststellen, dass kein Signal an einer der Kraftplatten vorhanden ist und deshalb das Signalkonditionierungs- bezw. Datenerfassungsteil des Systems rückstellen.

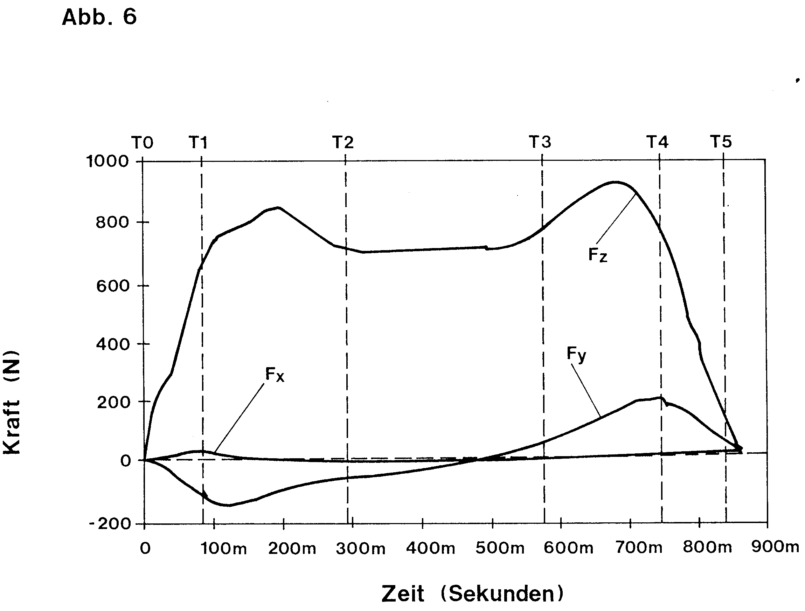

Die Diagramme in Abb. 5 betreffen nur die Z-Komponente, d.h. die während des Fussaufsetzens angewendete Vertikalkraft. In einem Mehrachsensystem stellt die Y-Achse die Laufrichtung dar, während die X-Achse quer zu dieser liegt und die Z-Achse senkrecht steht. Abb. 6 zeigt das Diagramm der drei Kräfte für einen einzelnen Fussaufschlag, zusammengesetzt aus den Informationen von den beiden Kraftplatten. Wie ersichtliche beginnt die YKraft als ein der Bewegungsachse entgenenwirkende Negativwert, und nimmt gegen T3 ab. In der Mittelauflage knapp vor T3 beim Uebersetzen auf die zweite Platte bekommt die Y-Kraft einen positiven Wert, was der Laufrichtung hilft. Die X-Komponente hat einen geringen positiven Wert anfänglich, am Ende des Schritts beim Aufprall und Abstoss, aber im allgemeinen einen geringen Negativwert während des übrigen Schritts, wodurch das Aufsetzen eines bestimmten Fusses auf die Kraftplatte angezeigt wird.

Ein zu berücksichtigender Faktor bei der Y-Achsenkraft ist die Reibung zwischen dem Tretmühlenriemen 4 und den Kraftplatten 1 und 2. Dies kann gelöst werden durch das Modellieren der Reibung zwischen der Kraftplatte und der Tretmühle, und das Subtrahieren dieser Kraft von den gesamten Y-Achsenkräften. Andere Lösungen sind ebenfalls anwendbar.

Wie aus den Diagrammen von Abbn. 6 und 5 ersichtlich, erfasst jede Kraftplatte ungefähr die halbe Auflagephase. Die erste Platte 1 empfängt etwa die erste Hälfte der Auflagephase mit einer Ueberschneidung, während die zweite Platte ungefähr die zweite Hälfte der Auflage bekommt. Um die auf die Kraftplatte wirkende Gesamtkraft zu berechnen, werden die Kräfte der Sensoren in jeder Platte summiert. Dies ergibt 100% der Gesamtauflage. Es ist unwichtig welcher Kraftanteil von einer Platte erfasst wird, da die Gesamtkraft immer 100% der Auflage darstellt. Ebenfalls unwichtig ist es, ob sich der Massenmittelpunkt der Testperson vor oder nach dem Spalt zwischen den beiden Kraftplatten befindet, wodurch die Zeitdauer auf der einen oder der anderen Platte geändert wird. Ein Schlürfen der Füsse beim Gehen hat im allgemeinen keinen Einfluss auf die Messung. Solange die Signale für das gleichzeitige Auftreten der beiden Füsse auf verschiedene Kraftplatten trennen lassen, können die entsprechenden Messungen durchgeführt werden.

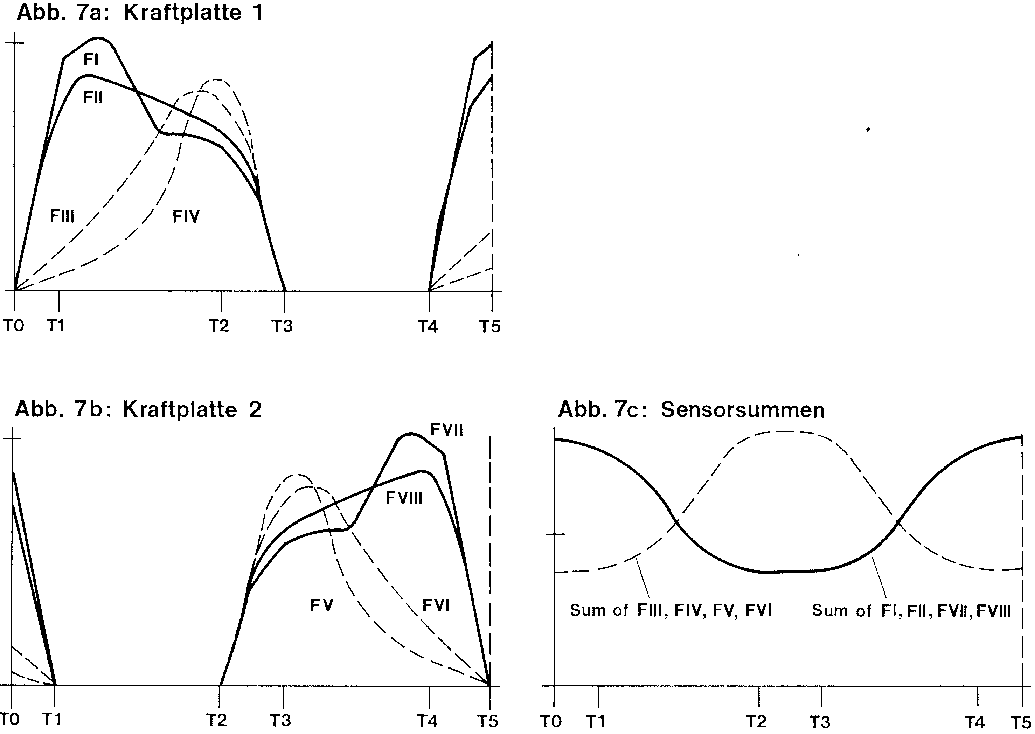

Um festzustellen, ob ein einziger Fuss auf beiden Platten steht oder zwei Füsse je auf einer Einzelplatte, überwacht das vorliegende System das Signal von jedem der acht Sensoren I bis VIII. Abb. 7A zeigt die Signale an den ersten vier Sensoren FI bis FIV der Kraftplatte 1, Abb. 7B die Signale an den zweiten vier Sensoren der zweiten Kraftplatte FV bis FVIII. Beim normalen Gang liegt der Aufsetzpunkt näher den beiden ersten Sensoren I und II an Platte 1, durch die Zeit T0 bis T1 dargestellt. Bei der Rückwärtsbewegung der Tretmühle bewegt sich das Fussgewicht dem Zentrum der vier Sensoren an der ersten Kraftplatte entgegen, und schliesslich der grössere Teil des Gewichts auf das zweite Sensorenpaar III und IV, wie zur Zeit T2 gezeigt. Mit der fortgesetzten Rückwärtsbewegung des Fusses über die beiden Platten, wird das Signal an III und IV um denselben Betrag vermindert als die Zunahme an den beiden ersten Sensoren der Platte 2, d.h. V and VI. Diese Phase beginnt mit T2.

Um T3 wird das Fussgewicht verschoben, so dass es hauptsächlich auf der zweiten Platte 2 liegt, mit den Kräften näher zu den Sensoren V und VI als dem hinteren Sensorenpaar VII und VIII. Das Fussgewicht wird dann auf der zweiten Platte zentriert und verschiebt sich anschliessend mehrheitlich auf den Hinterteil der Platte 2 mit einem Maximum an den Sensoren VII und VIII um die Zeit T4. Wenn der Fuss die Platte um T5 verlässt, gehen die Werte an allen vier Sensoren auf Platte 2 auf 0. Es ist zu bemerken, dass die ohne Buchstaben gekennzeichneten Teile der Diagramme in Abbn. 7A und 7B die Sensorwerte des anderen Fusses darstellen. Sie beginnen in Abb. 7A mit einem Fersenaufschlag um T4, und in Abb. 7B mit einem Zehe-ab-Segment zwischen T0 und T1.

Um zwischen einem Einzelfuss auf beiden Platten und zwei Füssen auf je einer Platte unterscheiden zu können, muss das Aufsetzen des Fusses auf zwei Platten genau bestimmt werden. Eine Prüfung der Diagramme in Abbn. 7A und 7B ergibt, dass es zwischen T2 und T3 eine Periode gibt, wo die Summe der vier anstossenden Sensoren FIII, FIV, FV und FVI grösser ist als die Summe der vier nichtanstossenden Sensoren FI, FII, FVII und FVIII. Die Summen werden in Abb. 7C gezeigt. Von der Zeit T0 bis T1 übersteigt die Summe der Signale an den vier nichtanstossenden Sensoren die Summe der vier anstossenden Sensoren. Um T0 ist ein Fuss wesentlich dem ersten Sensorensatz FI und FII in einer Fersen-ab-Lage anstossend, und der zweite Fuss befindet sich in einer Zehe-ab-Lage dem anderen Sensorensatz FVII und FVIII anstossend. Um T1 verschiebt sich das ganze Gewicht auf den Vorderfuss, den beiden ersten Sensoren FI und FII anstossend. Zwischen den Zeiten T2 und T3 übersteigen die Signale an den anstossenden Sensoren FIII, FIV, FV und FVI die Summe der Signalen an den vier Aussensensoren FI, FII, FVII und FVIII.

Eine einfache Bestimmung der Anwesenheit eines Einzelfusses auf beiden Platten wird erreicht, wenn die Summe der anstossenden Sensoren FIII bis FVI die Summe der Kräfte an den nichtanstossenden Sensoren Fi, FII, FVII, FVIII übersteigt. Unterschreitet die Summe der anstossenden Sensoren die Summe der nichtanstossenden Sensoren, dann ist ein Fuss auf der einen Platte oder ein Einzelfuss steht auf jeder Platte. Dies erlaubt eine Trennung der Daten von den Sensoren und den einzelnen Platten so, dass sie erfasst, gefolgt, erhalten sowie für jedes einzelne Fussaufsetzen angezeigt werden können.

Es ist zu bemerken, dass eine Tandemkraftplatte mit aktiven oder passiven Tretmühlen verwendet werden kann. Bei einer passiven Tretmühle wird der Riemen von der Testperson durch seine Gehbewegungen angetrieben. Bei einer aktiven Tretmühle wird der Riemen motorisch angetrieben. Die Ableitung der Kräfte sowie des Druckzentrums wie hier beschrieben gilt für verschiedene Geh- und Laufgeschwindigkeiten.

Da die Signale für jedes Fussaufsetzen sich individuell isolieren und verfolgen lassen, auch wenn beide Füsse eine der Kraftplatten berühren, bei der vorliegenden System wird das Druckzentrum unter Anwendung der folgenden Formel berechnet:

$$A_{x1} = a(F_{23} - F_{14})/F_{T1} \qquad A_{y1} = b(F_{12} - F_{34})/F_{T1}$$
$$A_{x2} = a(F_{67} - F_{58})/F_{T2} \qquad A_{y2} = b(F_{56} - F_{78})/F_{T2}$$

wo

$A_{xp}$ = x-Achse Druckzentrumslage für Kraftplatte P
$A_{yp}$ = y-Achse Druckzentrumslage für Kraftplatte P
$F_{Tp}$ = Gesamtkraft von allen 4 Sensoren für Platte P
$F_{mm}$ = Summe der Messwandlerkräfte für Messwandler "m", "n"
2a = Abstand zwischen Messwandlern I, II, III, IV; V, VI, VII, VIII
2b = Abstand zwischen Messwandlern I, IV; II, III; V, VII; VI, VIII

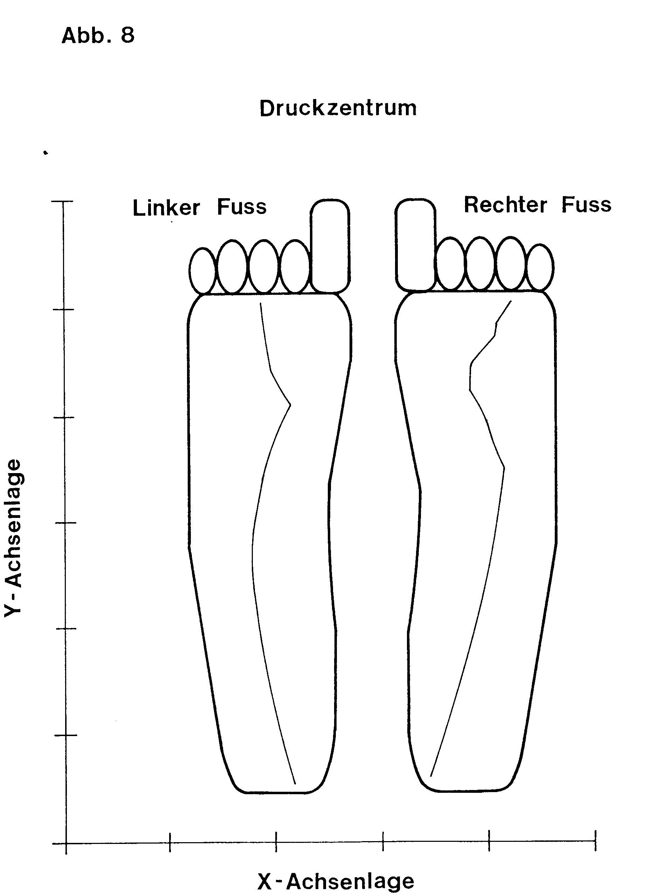

Für jeden Abtastpunkt lässt sich das Druckzentrum der einzelnen Füsse auf den Tandemkraftplatten berechnen. Die Geschwindigkeit der Tretmühle gemäss Geschwindigkeitsensor 8 wird von der Fussbewegung an den Kraftplatten subtrahiert, um das eigentliche Druckzentrum für jedes Fussaufsetzen zu erhalten. Abb. 8 zeigt das resultierernde Diagramm des Druckzentrums dem linken und rechten Fuss entlang im Phantom.

Hinsichtlich Abb. 8 gilt das Gitter als die Summe der beiden Platten so, dass das Druckzentrum während des ganzen Schritts verfolgt wird.

Obwohl die vorliegende Erfindung im Detail erläutert und illustriert wurde, ist dies selbständig nur als Veranschaulichung und Beispiel zu verstehen. Zwar sind vier Sensoren pro Kraftplatte gezeigt, aber zusätzliche Sensoren können auch vorgesehen werden. Die Verwendung von vier Sensoren erlaubt den Einsatz von handelsüblichen Kraftplatten. Ebenfalls dürfen mehr als zwei Kraftplatten verwendet werden, z.B. vier, wobei Kraftplatten 1 und 2 hälftig geteilt werden. Auch ist zu beachten, das die beschriebene Erfindung zwar für den Einsatz im Zusammenhang mit einer Tretmühle gedacht ist, aber eine grössere Zahl von Kraftplatten lassen sich auch als fester Gehweg verwenden, da die Signalverarbeitung der vorliegenden Erfindung eine getrennte Verarbeitung jedes Fussaufsetzens erlaubt, gleich ob ein Plattenpaar überbrückt wird oder ein Fuss die entsprechende Platte individuell berührt. Der Umfang und die Wesensart der vorliegenden Erfindung werden lediglich durch die Bedingungen der nachfolgenden Ansprüchen begrenzt.

**Patentansprüche**

1. Ein Fussaufschlagmesssystem bestehend aus: einer ersten und einer zweiten Platte in Tandemanordnung bezüglich der Gehrichtung einer Testperson auf besagtem System; erstes und zweites Sensormittel zur Erfassung von Kräften an einer zugehörigen Platte; ein Mittel zur Bestimmung aus dem ersten und zweiten Sensormitteln, ob ein Einzelfuss auf beiden Platten oder ein Fuss auf jeder Platte steht; und ein Mittel zur Verarbeitung der Signale von besagten erstem und zweitem Sensormittel als Funktion besagter Bestimmung, um individuelles Fussaufsetzen zu messen.

2. Ein System gemäss Anspruch 1, wobei jedes besagte Sensormittel mindestens zwei Messwandler enthält, entlang besagter Gehrichtung verteilt; und besagtes Bestimmungsmittel erfasst einen Einzelfuss auf beiden Platten, wenn die Summe der Kräfte an einem ersten Paar erster Messwandler, von jeder Platte einer und aneinander stossend, die Summe der Kräfte an einem zweiten Paar erster Messwandler übertrifft, von jeder Platte einer und durch besagtes erstes Paar erster Messwandler getrennt.

3. Ein System gemäss Anspruch 2, wobei jedes besagte Sensormittel mindestens zwei Zweitmesswandler umfasst, entlang besagter Gehrichtung verteilt sowie quer zur besagten Gehrichtung von einem entsprechenden Erstmesswandler verteilt; und besagtes Bestimmungsmittel einen Einzelfuss auf beiden Platten erfasst, wenn die Summe der Kräfte an Erstpaaren von ersten und zweiten Messwandlern, einer von jeder Platte und aneinander stossend, die Summe der Kräfte an Zweitpaaren von ersten und zweiten Messwandlern übertrifft, einer von jeder Platte und durch besagte Erstpaare von ersten und zweiten Messwandlern getrennt.

4. Ein System gemäss Anspruch 3, wobei besagtes Verarbeitungsmittel für jedes Fussaufsetzen aus den Kräften an besagten ersten und zweiten Messwandlerpaaren ein Druckzentrum bestimmt.

5. Ein System gemäss Anspruch 1, wobei besagtes Sensormittel mindestens zwei entlang besagter Gehrichtung verteilte Erstmesswandler umfasst, sowie mindestens zwei entlang besagter Gehrichtung verteilte Zweitmesswandler, gegenüber besagter Gehrichtung quer verteilt von einem entsprechenden Erstmesswandler aus, und besagtes Verarbeitungsmittel für jedes Fussaufsetzen aus den Kräften an besagten ersten und zweiten Messwandlerpaaren bestimmt.

6. Ein System gemäss Anspruch 5, wobei besagtes System umfasst:
einen Tretmühlenriemen über besagten Platten;
einen Sensor zur Erfassung der Geschwindigkeit besagten Tretmühlenriemens; und
besagtes Verarbeitungsmittel, das für jedes Fussaufsetzen aus den Kräften an besagten ersten und zweiten Messwandlerpaaren sowie der Geschwindigkeit besagten Tretmühlenriemens ein Druckzentrum bestimmt.

7. Ein System gemäss Anspruch 1, einschliesslich eines Tretmühlenriemens über besagten Platten.

8. Ein System gemäss Anspruch 7, einschliesslich Mittel zur Regelung der Geschwindigkeit besagten Tretmühlenriemens, damit die Testperson wesentlich zentriert über besagten, entlang der besagten Gehrichtung verteilten Platten bleibt.

9. Ein System gemäss Anspruch 1, einschliesslich Mittel zur individuellen Rückstellung besagten Sensormittels bei Nichterfassung einer Kraft durch das Sensormittel während eines Messzyklus.

10. Ein System gemäss Anspruch 1, wobei besagtes Verarbeitungsmittel besagte Signale von besagten ersten und zweiten Sensormitteln kombiniert, wenn ein Fuss auf beiden Platten bestimmt wird, und besagte Signale von besagten ersten und zweiten Sensormittel trennt, wenn ein Fuss auf jeder Platte festgestellt wird.

11. Ein System gemäss Anspruch 1, wobei jedes besagte Sensormittel mindestens zwei besagter Gehrichtung entlang verteilte Erstmesswandler enthält, und besagte Messwandler Kräfte der Parallelachse entlang erfassen.

12. Ein System gemäss Anspruch 1, wobei jedes besagte Sensormittel mindestens zwei besagter Gehrichtung entlang verteilte Erstmesswandler enthält, und jeder Messwandler Kräfte entlang einer von drei Orthogonalachsen erfasst.

13. Eine Methode zur individuellen Verarbeitung von Signalen für fortlaufendes Fussaufsetzen in einem System mit zwei Kraftplatten in Tandemanordnung einer Gehrichtung entlang, und einem ersten und zweiten Sensormittel für eine entsprechende Platte, wobei
von dem ersten und zweiten Sensor bestimmt wird, ob die Kraft an besagten zwei Platten näher zu anstossenden oder nichtanstossenden Kanten besagter Platten entlang besagter Gehrichtung liegt; und
besagte Signale von besagten ersten und zweiten Sensormitteln für ein einzelnes Fussaufsetzen kombiniert werden, wenn näher zu anstossenden Kanten festgestellt wird, und besagte Signale vom besagten ersten und zweiten Sensormittel getrennt werden als ein Paar Fussaufschläge bei Bestimmung nahe den nichtanstossenden Kanten.

**Abb. 1a**

Abb. 1b

Abb. 2

Datenerfassungscomputer

Daten – und Programm – speicher

Disketten – laufwerke

Druck / Plotter – Ausgänge

Datenerfassung

CPU

Bildschirm – Adapter

Drucker

Plotter

Tastatur

Bildschirm – monitor

Signal - konditionie- rung

Instrumentier. Tretmühle

Geschwindikeitssensor

Schwingungssensor

Kraftplatte 1

Kraftplatte 2

10

Abb. 3a

Abb. 3b

Abb. 3c

Abb. 4a

Abb. 4b

EP 0 603 115 A2

Abb. 5d: KP1+KP2 – rechter Fuss

Abb. 5e: KP1+KP2 – linker Fuss

Abb. 5a: KP1 – beide Füsse

Abb. 5b: KP2 – beide Füsse

Abb. 5c: KP1+KP2 – beide Füsse

— Zwei Füsse auf beiden Platten

— Ein Fuss auf beiden Platten

Abb. 6

EP 0 603 115 A2

Abb. 7a: Kraftplatte 1

Abb. 7b: Kraftplatte 2

Abb. 7c: Sensorsummen

Sum of FI, FII, FVII, FVIII

Sum of FIII, FIV, FV, FVI

**Abb. 8**

Druckzentrum

Linker Fuss     Rechter Fuss

Y - Achsenlage

X - Achsenlage